# EUROPÄISCHE PATENTSCHRIFT

(11) **EP 1 425 048 B1**
(45) Veröffentlichungstag und Bekanntmachung des Hinweises auf die Patenterteilung: **23.04.2008**
(21) Anmeldenummer: 02758445.7
(22) Anmeldetag: 08.08.2002
(51) Int. Cl.: A61L 2/20, B65B 55/10

(54) **STERILISIERVORRICHTUNG MIT H2O2-VERDAMPFER**
STERILIZER COMPRISING A H2O2 EVAPORATOR
DISPOSITIF DE STERILISATION A EVAPORATEUR H2O2

(30) Priorität: 17.09.2001 DE 10145818
(43) Veröffentlichungstag der Anmeldung: 09.06.2004
(73) Patentinhaber: KHS AG, 44143 Dortmund (DE)
(72) Erfinder: GROSSMANN, Holger, 21109 Hamburg (DE); HEROLD, Thomas, 23898 Duvensee (DE)
(86) Internationale Anmeldenummer: PCT/EP2002/008860
(87) Internationale Veröffentlichungsnummer: WO 2003/024492

(56) Entgegenhaltungen:
- EP-A- 0 321 908
- WO-A-99/30747
- US-A- 4 797 255
- US-A- 5 152 968
- US-A- 5 873 181

## Beschreibung

Die Erfindung betrifft eine Vorrichtung nach dem Oberbegriff des Anspruches 1.

Eine derartige Vorrichtung wird in der US-PS 4 797 255 beschrieben. Hier ist ein kastenartiger Verdampfungsraum vorgesehen, der kopfseitig mit hieran hängend angebrachten Düsen für die Zufuhr von H₂O₂ ausgerüstet ist. Im Innern des Verdampfungsraumes befindet sich ein Zufuhrrohr für Luft mit einer Vielzahl von Luftöffnungen. Außerdem weist der Verdampfungsraum unter anderem eine Heizplatte auf.

Solche Vorrichtungen sterilisieren im Allgemeinen Getränkebehälter, wie z. B. Kunststoffflaschen von innen oder ggf. auch von außen mit einem H₂O₂-Dampf enthaltenden Luftstrom. Dieser weist eine zur Verdampfung des H₂O₂ erforderliche höhere Temperatur auf. Überstreicht er die kühleren Behälteroberflächen, so kondensiert dort H₂O₂ als feiner Film auf. Dieser sorgt für eine gleichmäßige Sterilisierung der Oberfläche im Gegensatz zu älteren Konstruktionen, bei denen die Oberflächen direkt mit feinen Tröpfchen besprüht werden.

Bei gattungsgemäßen Vorrichtungen erfolgt die Verdampfung des H₂O₂, das üblicherweise in wässriger Lösung geeigneter Konzentration vorliegt, das also zusammen mit dem Wasser verdampft wird, in einem Verdampfungsraum eines H₂O₂-Verdampfers aus dem mit durchgeblasener Luft der H₂O₂-Dampf dem zu sterilisierenden Behälter zugeführt wird. Dabei wird üblicherweise ein Verdampfer pro Behälterplatz vorgesehen und taktweise zum Sterilisieren aufeinander folgender Behälter eingesetzt.

Bei bekannten Konstruktionen, wie sie aus DE 32 35 476 C2, DE 35 40 161 C2 und DE 39 00 448 C2 (Fig. 2) bekannt sind, wird H₂O₂ über eine Zweistoffdüse in den Verdampfungsraum gesprüht. Die Sprühtröpfchen gelangen auf die beheizten Flächen und verdampfen dort. Diese Methode ist anderen Verfahren überlegen, bei denen die Verdampfung in Heißluft erfolgt, da die Wärmekapazität von Luft der von Feststoffen (Wandmaterial) weit unterlegen ist.

Eine ähnliche Konstruktion ist aus DE 197 04 639 C2 bekannt. Dort wird mit einer Einstoffdüse in einem auf andere Weise zugeführten Luftstrom im Verdampfungsraum auf Wandflächen gesprüht, die jedoch aus speziellen Gründen unterhalb der Verdampfungstemperatur gehalten werden. Erst im weiteren Luftstrom wird bei höheren Wandtemperaturen verdampft.

Die US-A-4 797 255 offenbart eine Vorrichtung und ein Verfahren bei denen H₂O₂ in Form von Tropfen mit einem Durchmesser von 1 bis 3 mm auf eine Verdampferfläche einer Heizvorrichtung zur sofortigen Verdampfung getropft wird. Nach der Verdampfung erfolgt die Vermischung mit Luft.

Auch in der EP-A-0 321 908 wird Luft gegen bereits verdampftes H₂O₂ eingespeist.

Nachteilig bei dem geschilderten Stand der Technik ist die Verwendung von Einstoff- bzw. Zweistoffdüsen, die in ihrer aus dem Stand der Technik bekannten Bauart mit sehr engen Kanälen arbeiten, die den Verdüsungseffekt bewirken. Verdüsung in sehr kleine Tröpfchen ist jedoch notwendig, um anschließend eine rasche Verdampfung zu erzielen.

Bei den bekannten Konstruktionen neigen die Düsen zum raschen Verstopfen. Bedingt ist dies durch üblicherweise in H₂O₂-/Wasserlösungen enthaltene Stabilisatorsalze, die zum Ausfallen und zur Bildung kleiner Körnchen neigen, welche die feinen Düsenkanäle verstopfen. Auch mit vorgeschalteten Filtern ist diesem Effekt nur unzureichend beizukommen.

Die Aufgabe der vorliegenden Erfindung besteht darin, eine gattungsgemäße Vorrichtung dahingehend weiter zu bilden, dass ein rasches Verdampfen der Flüssigkeit erreicht wird und Verstopfungen vermieden werden.

Diese Aufgabe wird mit den Merkmalen des Anspruches 1 gelöst.

Erfindungsgemäß erfolgt die Verdüsung mit einer Düseneinrichtung in der ein Luftstrahl und ein H₂O₂-Strahl getrennt voneinander erzeugt werden. Sie treffen sich im freien Raum, wobei an der Kreuzungsstelle der Luftstrahl den H₂O₂-Strahl zu feinen Tröpfchen zerreißt. In der H₂O₂-Düse findet keine Verdüsung in kleine Tröpfchen statt, sondern es wird ein massiver Strahl erzeugt. Die dazu erforderlichen Düsenkanäle weisen derart große Querschnitte auf, dass sie von Salzkristallen nicht verstopft werden können.

Vorteilhaft sind die Merkmale des Anspruches 2 vorgesehen. Auf diese Weise kann nach der Verdampfung im Verdampfungsraum in den weiterführenden Kanälen erforderlichenfalls nachverdampft werden, falls noch nicht verdampfte Tröpfchen im Luftstrahl sind. Außerdem kann in den Kanälen bis zum Auslass die Temperatur auf den gewünschten Wert stabilisiert werden.

In der Zeichnung ist die Erfindung beispielsweise und schematisch dargestellt. Es zeigen:
- **Fig. 1**: einen Schnitt nach Linie 1-1 in Fig. 2 durch einen H₂O₂-Verdampfer und
- **Fig. 2**: einen Schnitt nach Linie 2-2 in Fig. 1.

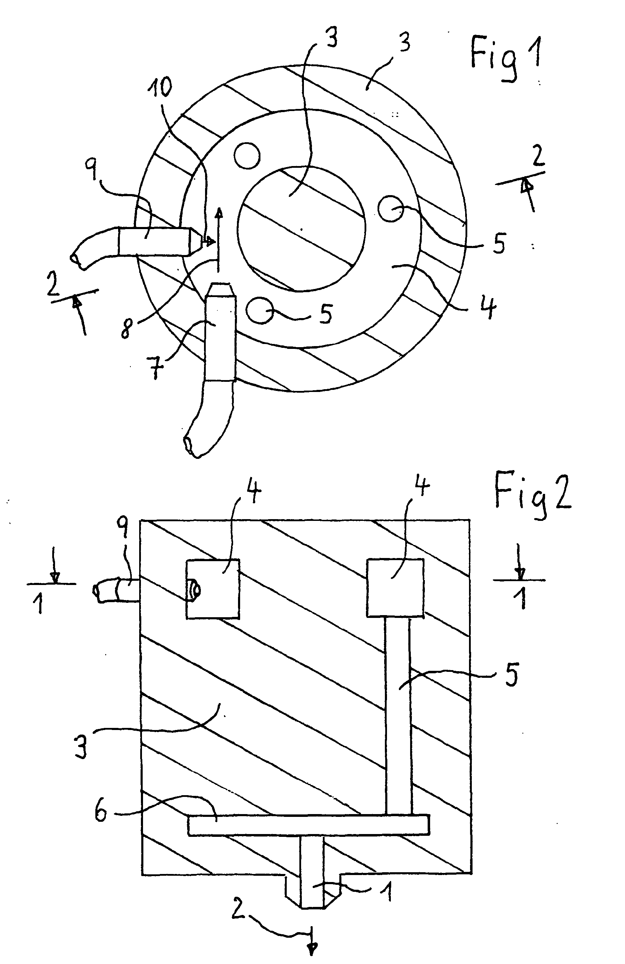

Der in den Figuren 1 und 2 dargestellte H₂O₂-Verdampfer ist Teil einer Vorrichtung zum Sterilisieren von Getränkebehältern. An seinem Auslaß 1 erzeugt er eine Strömung 2 bestehend aus Luft mit einem Anteil von H₂O₂-Dampf sowie gegebenenfalls Wasserdampf, falls H₂O₂ in der üblichen wässrigen Lösung geeigneter Konzentration verwendet wird. Der Auslaß 2 kann in bekannter Weise z.B. über dem Einlaß einer Flasche oder eines sonstigen Getränkebehälters angeordnet sein, um den Innenraum zu sterilisieren.

Der dargestellte Verdampfer weist einen massiven, in nicht dargestellter Weise mit Trennflächen auseinandernehmbaren Gehäuseblock 3 auf, in dessen dem Auslaß 1 gegenüberliegenden Endbereich ein Ringkanal 4 ausgebildet ist. Der Ringkanal 4 ist mit im Ausführungsbeispiel drei parallel zur Achse des Auslaßes 1 angeordneten Kanälen 5 und einem Sammelraum 6, in den alle drei Kanäle 5 münden, mit dem Auslaß 1 verbunden.

Der Gehäuseblock 3 ist mit nicht dargestellten Einrichtungen auf eine zum Verdampfen des H₂O₂ geeignete Temperatur geheizt, so daß auch seine sämtlichen Innenwände entsprechende Temperatur annehmen.

Wie Figur 1 zeigt, mündet in den Ringkanal 4 tangential eine Luftdüse 7, die einen gebündelten Luftstrahl 8 erzeugt, der tangential einläuft, eine Kreisströmung im Ringkanal 4 erzeugt und von diesem über die Kanäle 5 und den Sammelraum 6 zum Auslaß 1 strömt.

Eine H₂O₂-Düse 9 ist im Gehäuseblock 3 derart angeordnet, daß sie einen Flüssigkeitsstrahl 10 quer zum Luftstrahl 8 im Abstand zu diesem erzeugt, und zwar derart, daß der Flüssigkeitsstrahl 10 den Luftstrahl 8 im freien Raum innerhalb des Ringkanales 4 trifft.

Die H₂O₂-Düse 9 ist derart ausgebildet, daß sie einen massiven Flüssigkeitsstrahl etwa im Durchmesserbereich einiger Zehntel Millimeter erzeugt. Im H₂O₂ bzw. im üblicher Weise verwendeten H₂O₂-/Wassergemisch vorhandene Salzkristalle oder sonstige bei üblichen Sprühdüsen zur Verstopfung führende Feststoffe werden in einem Flüssigkeitsstrahl dieser Dicke ohne weiteres durchgelassen ohne, daß die H₂O₂-Düse 9 verstopfen kann.

Der Flüssigkeitsstrahl 10 trifft den Luftstrahl 8 in einem Kreuzungspunkt und wird von diesem durch die mit geeigneter Strömungsgeschwindigkeit strömende Luft mitgerissen und zu Tröpfchen zerrissen. Der so erzeugte Sprühnebel aus feinen Tröpfchen läuft mit dem von dem tangential einströmenden Luftstrahl 8 erzeugten Luftstrahl kreisförmig um den Ringkanal um. Dabei werden die am Kreuzungspunkt der Strahlen erzeugten feinen Flüssigkeitströpfchen durch Zentrifugalkraft überwiegend nach außen auf die Umfangswand des Ringkanals 4 transportiert, um sich dort abzusetzen.

Die Tröpfchen verdampfen dort. Die erzeugte Mischung von Luft und H₂O₂-Dampf gelangt anschließend durch die ebenfalls noch beheizten Kanäle 5 zum Auslaß 1. In den Kanälen 5 können evtl. noch vorhandene Flüssigkeitströpfchen nachverdampfen und es wird eine Wiederkondensation im Verdampfer verhindert.

In nicht dargestellter Ausführung kann anstelle des Ringkanals 4 auch ein sonstiger Verdampfungsraum vorgesehen sein, in dem das Versprühen des H₂O₂ durch gekreuzte Strahlen von Luft und Flüssigkeit erfolgt. Vorzugsweise kann dieser Raum dabei eine runde Umfangswand haben und der Luftstrahl 8 tangential, wie in Figur 1 dargestellt, eingeblasen werden, um ähnliche Effekte wie beim Ringkanal zu erzeugen.

Anstelle der geraden Kanäle 5 können auch auf gewundenen Wegen, zum Beispiel spiralförmig verlegte Kanäle zwischen dem Verdampfungsraum 4 und dem Auslaß 1 vorgesehen sein, um auf längerem Wege eine gute Nachheizung zu bewirken.

## Patentansprüche

1. Vorrichtung zum Sterilisieren von Getränkebehältern durch Strömungsbeaufschlagung mit einem Gemisch aus Luft und H₂O₂-Dampf, mit einem H₂O₂-Verdampfer, der einen luftdurchströmten Verdampfungsraum (4) mit beheizten Wänden aufweist, gegen die H₂O₂ von einer Düseneinrichtung (7, 9) gesprüht wird, wobei die Düseneinrichtung (7, 9) eine einen Luftstrahl (8) einblasende Luftdüse (7) und eine H₂O₂-Düse (9) aufweist, die zur Erzeugung eines H₂O₂-Strahles (10) im Abstand zum Luftstrahl (8) und auf diesen gerichtet ausgebildet ist, **dadurch gekennzeichnet, dass**
- der Verdampfungsraum (4) ringförmig ausgebildet und die Luftdüse (7) tangential in diesen einblasend ausgebildet ist, wobei
- die H₂O₂-Düse (9) einen Flüssigkeitsstrahl (10) quer zum Luftstrahl (8) erzeugt, und zwar derart, dass der Flüssigkeitsstrahl (10) den Luftstrahl (8) im freien Raum innerhalb des Ringkanales (4) trifft, so dass
- Tröpfchen mit dem Luftstrahl (8) im Kreis durch den Ringraum geblasen werden und sich durch Zentrifugalkraft großflächig auf der Außenfläche des Ringraumes niedersetzen, wo sie verdampfen.

2. Vorrichtung nach Anspruch 1, **dadurch gekennzeichnet, dass** der Verdampfungsraum (4) mit einem Auslass (1) des Verdampfers über beheizte Kanäle (5) verbunden ist.

## Claims

1. Apparatus for sterilising beverage containers by subjecting them to a flow made up of air and H₂O₂ vapour, the said apparatus including an H₂O₂ evaporator that includes an evaporation chamber (4), which is traversed by air, with heated walls against which H₂O₂ is sprayed from a nozzle device (7, 9), wherein the nozzle device (7, 9) includes an air nozzle (7), which blows in an air jet (8), and an H₂O₂ nozzle (9), which is designed to generate an H₂O₂ jet (10) at a spacing from the air jet (8) and is directed towards the said air jet, **characterised in that**
- the evaporation chamber (4) is ring-shaped and the air nozzle (7) is designed to blow into the said vaporisation chamber in a tangential manner, whereby
- the H₂O₂ nozzle (9) generates a liquid jet (10) transversely relative to the air jet (8) such that the liquid jet (10) contacts the air jet (8) in the free space inside the annular duct (4), such that
- droplets are blown with the air jet (8) in a circular manner through the annular chamber and are deposited by centrifugal force over a large surface area on the outside surface of the annular chamber, where they evaporate.

2. Apparatus according to claim 1, **characterised in that** the evaporation chamber (4) is connected to an outlet (1) of the evaporator by means of heated ducts (5).

## Revendications

1. Dispositif servant à stériliser des contenants pour boissons en les soumettant à l'écoulement d'un mélange constitué d'air et de vapeur de H₂O₂, comprenant un évaporateur de H₂O₂ présentant une chambre d'évaporation (4) traversée par un courant d'air et pourvue de parois chauffées contre lesquelles du H₂O₂ est pulvérisé par un dispositif à buse (7, 9), le dispositif à buse (7, 9) présentant une buse d'air (7) insufflant un jet d'air (8) et une buse de H₂O₂ conçue pour générer un jet de H₂O₂ (10) à distance du jet d'air (8) et dirigé sur celui-ci, **caractérisé en ce que**
la chambre d'évaporation (4) est annulaire et la buse d'air (7) est conçue pour insuffler de l'air de façon tangentielle dans celle-ci,
la buse de H₂O₂ (9) produisant un jet de liquide (10) perpendiculairement au jet d'air (8), de telle sorte que le jet de liquide (10) touche le jet d'air (8) dans l'espace libre existant à l'intérieur du canal annulaire (4), de telle sorte que
des gouttelettes soient soufflées avec le jet d'air (8), de façon circulaire à travers l'espace annulaire et se déposent, du fait de la force centrifuge, sur une grande étendue de la surface externe de l'espace annulaire, où elles s'évaporent.

2. Dispositif selon la revendication 1, **caractérisé en ce que** la chambre d'évaporation (4) est reliée à une sortie (1) de l'évaporateur, par l'intermédiaire de canaux (5) chauffés.
